(19) Europäisches Patentamt
European Patent Office
Office européen des brevets

(11) Publication number: **0 408 384 A1**

# (12) EUROPEAN PATENT APPLICATION

(21) Application number: **90307686.7**

(51) Int. Cl.[5]: **C07C 237/06**, A61K 31/22

(22) Date of filing: **13.07.90**

The title of the invention has been amended (Guidelines for Examination in the EPO, A-III, 7.3).

(30) Priority: **13.07.89 GB 8916071**

(43) Date of publication of application: **16.01.91 Bulletin 91/03**

(84) Designated Contracting States: **AT BE CH DE DK ES FR GB GR IT LI LU NL SE**

(71) Applicant: **NATIONAL RESEARCH DEVELOPMENT CORPORATION**
**101 Newington Causeway**
**London SE1 6BU(GB)**

(72) Inventor: **Creighton, Andrew Malcolm**
**68 Millway**
**Mill Hill, London NW7 3QY(GB)**
Inventor: **Jeffery, William Anthony**
**16 East Street, Tollesbury**
**Maldon, Essex CM9 8QD(GB)**

(74) Representative: **Stephenson, Gerald Frederick**
**Patent Department National Research Development Corporation 101 Newington Causeway**
**London SE1 6BU(GB)**

## (54) N,N'-dicarboxymethyl, N,N'-dicarbamoylmethyl diaminoalkane derivatives and their use in pharmaceutical compositions.

(57) Compounds of formula (II):

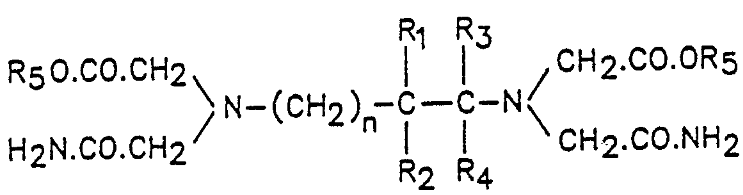

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-5}$ alkyl groups substituted by a hydroxy group or by a $C_{1-6}$ alkoxy group, or one of $R_1$ and $R_2$ and one of $R_3$ and $R_4$ is hydrogen and the others together are a trimethylene, tetramethylene or pentamethylene bridging group, and $R_5$ is a group such that under physiological conditions $R_5OH$ undergoes elimination with the formation of a 3,5-dioxopiperazinyl ring, but with the provisos that (a) when n = 0 the combinations $R_1 = R_2 = R_3 = R_4 = H$ and $R_1 = R_2 = R_3 = H$, $R_4$ = methyl are excluded and (b) when n is 0, $R_1$ and $R_3$ are each hydrogen, and each of $R_2$ and $R_4$ is other than hydrogen the compound is in other than the meso or erythro configuration, and salts thereof formed with a physiologically acceptable inorganic or organic acid, are of use in therapy, particularly as cardioprotective agents.

# PHARMACEUTICAL COMPOSITIONS

This invention relates to pharmaceutical compounds and to compositions containing them, being primarily concerned with substances of use as cardioprotective agents and in certain other protective roles.

Certain bis-dioxopiperazines of formula (I) are cytotoxic and have been used in the treatment of cancer. Thus UK Patent 1,234,935 describes the compounds of formula (I) having R = $CH_3$ and R′ = R″ = H (as the dl , d and l isomers); R = R′ = R″ = H; R = R′ = $CH_3$ and R″ = H (as the meso isomer); and R + R′ = $-CH_2CH_2-$ and R″ = H. Of these the first named compound has proved to be of most value although a further compound of formula (I) having R = R′ = H and

R″ = 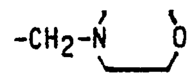

has also been used in treating cancer.

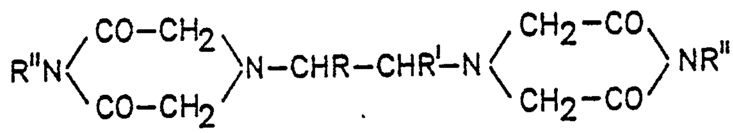

(I)

Studies have been reported by various authors on the chelating properties of these bis-dioxopiperazines and the use in the treatment of lead poisoning has been proposed by Wittig and Hultsch, Int. Arch. Occup. Environ. Health, 1981, 48 , 89, for the compound of formula (I) having R = R′ = H and R″ = $CH_3$ and by May et al , Agents and Actions, 1984, 15 , 448 and Willes and Williams, Plzen. Lek. Sborn., 1985, 49 , 113, for the compound having R = H and R′ = $C_2H_5$ in the dl form.

In Research Communications in Chemical Pathology and Pharmacology, 1985, 48, 39, Herman et al report tests on the protective effect against acute daunorubicin toxicity of a range of bis-dioxopiperazines of formula (I). They conclude that although the compound bimolane (R = R′ = H and

R″ = 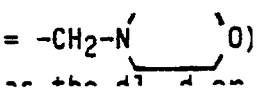)

and the compound having R = $CH_3$ and R′ = R″ = H (as the dl , d or l isomer) give protection against the lethal effects of daunorubicin, the remainder of the compounds tested (R = R′ = R″ = H; R = R′ = H and R″ = $CH_3$; R = R″ = $CH_3$ and R′ = H ( l ); R = R′ = $CH_3$ and R″ = H ( meso ); R = $C_2H_5$ and R′ = R″ = H ( dl ); R = $CH_3$, R′ = $C_2H_5$ and R″ = H ( dl-erythro ); and R + R′ = $-CH_2-CH_2-$ and R″ = H; as well as the compound in which -CHR-CHR′- is replaced by $-(CH_2)_3-$ and the ring opened bis-diacid diamide compound having R = $CH_3$ and R′ = H) all showed either no protective activity or only minimal protective activity.

It is the case that all of the bis-dioxopiperazines identified in this paper as exhibiting a useful level of cardioprotection against daunorubicin toxicity are cytotoxic. We have now found that despite the indications to the contrary in the paper, certain pro-drugs of various bis-dioxopiperazines which are substantially non-cytotoxic are of interest as cardioprotective agents, as well as in other roles. These non-cytotoxic bis-dioxopiperazine pro-drugs are of interest for providing protection against the cardiotoxic effects of various anthracycline drugs but particularly doxorubicin (adriamycin). In this context it is relevant that, in addition to the comments in the Herman et al Research Communications in Chemical Pathology and Pharmacology paper it is indicated by Herman et al in Advances in Pharmacology and Chemotherapy, 1982, 19 , 249 that even the cardioprotective compound ICRF 159, which is the dl isomer of the compound of formula (I) having R = $CH_3$ and R = R′ = H, is consistently more effective in reducing high dose daunorubicin toxicity than doxorubicin toxicity.

Accordingly the present invention comprises a compound of formula (II):

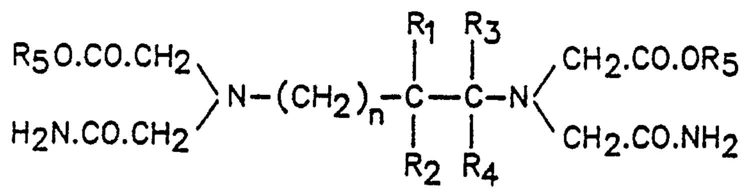

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-6}$ alkyl groups substituted by a hydroxy group or by a $C_{1-6}$ alkoxy group, or one of $R_1$ and $R_2$ and one of $R_3$ and $R_4$ is hydrogen and the others together are a trimethylene, tetramethylene or pentamethylene bridging group, and $R_5$ is a group such that under physiological conditions $R_5OH$ undergoes elimination with the formation of a 3,5-dioxopiperazinyl ring, but with the provisos that (a) when n = 0 the combinations $R_1$ = $R_2$ = $R_3$ = $R_4$ = H and $R_1$ = $R_2$ = $R_3$ = H, $R_4$ = methyl are excluded and (b) when n is 0, $R_1$ and $R_3$ are each hydrogen, and each of $R_2$ and $R_4$ is other than hydrogen the compound is in other than the meso or erythro configuration, and salts thereof formed with a physiologically acceptable inorganic or organic acid.

It will be appreciated that the above provisos also extend to equivalent combinations so that, for example, a compound having n = 0, $R_1$ = $CH_3$ and $R_2$ = $R_3$ = $R_4$ = $R_5$ = H is the same as that having n = 0, $R_1$ = $R_2$ = $R_3$ = $R_5$ = H and $R_4$ = $CH_3$, and the proviso applying to the compounds in which n is 0, $R_1$ and $R_3$ are each hydrogen and each of $R_2$ and $R_4$ is other than hydrogen applies equally to the situation in which n is 0, $R_2$ and $R_4$ are each hydrogen and each of $R_1$ and $R_3$ is other than hydrogen.

The pro-drug compounds (II) which are the subject of the present invention do not undergo simple hydrolysis of the two similar ester groups in vivo which would result in a non-cyclic diamide diacid compound of formula (IV) as described hereinafter but instead eliminate two $R_5OH$ molecules with the formation of a compound of formula (III) containing two dioxopiperazine rings

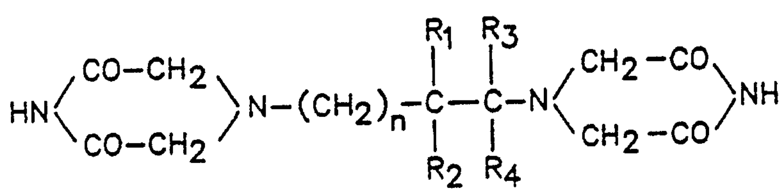

(III)

In contrast with bimolane and the compound of formula (I) having R = $CH_3$ and $R'$ = $R''$ = H, the compounds (III) produced in vivo from the pro-drug compounds (II) of the present invention have the particular advantage of being substantially non-cytotoxic. The compounds (II) are thus distinguished from the related pro-drugs which are the subject of UK Patent GB 2173195 and which are converted in vivo to cytotoxic compounds having a formula (I) as given hereinbefore with $R''$ being hydrogen and $R$ and $R'$ being as defined in that patent.

In the pro-drugs (II) of the present invention the central grouping in the molecule has the form:

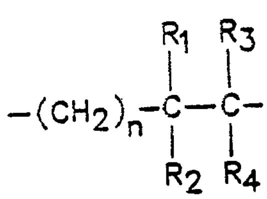

Although n may be 2 or more especially 1, each of $R_1$ to $R_4$ then conveniently being hydrogen, it is

preferred that n is 0, in which latter case the grouping will be of the form:

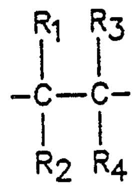

As indicated, $R_1$, $R_2$, $R_3$ and $R_4$ may be hydrogen or an unsubstituted acyclic $C_{1-6}$ aliphatic hydrocarbon group or a $C_{1-6}$ alkyl group substituted by a hydroxy group or a $C_{1-6}$ alkoxy group, subject to the exclusion of two specific compounds in the case when n = 0 which are converted in vivo to cytotoxic compounds (III). The term acyclic aliphatic hydrocarbon group is used herein to include both branched and especially straight chain groups. The group may be unsaturated or especially saturated, conveniently containing one double or triple bond in the former case. Thus, in particular, unsubstituted groups may be alkenyl, alkynyl or particularly alkyl groups (the terms alkyl, alkenyl and alkynyl are used throughout this specification to include both straight and branched groups). The aliphatic hydrocarbon groups conveniently contain a maximum of four or especially three carbon atoms, preferred groups therefore being $C_1$-$C_4$ or $C_1$-$C_3$ alkyl groups and $C_2$-$C_4$ or $C_2$-$C_3$ alkenyl and alkynyl groups.

As regards the substituted $C_{1-6}$ alkyl groups $R_1$ to $R_4$, these may be branched or especially straight chain alkyl groups substituted, particularly terminally, by a hydroxy group or particularly an alkoxy group. Conveniently the groups are of 1 to 3 or 1 to 4 carbon atoms, substituted ethyl and particularly substituted methyl groups being of most interest. Preferred alkoxy group substituents similarly contain 1 to 3 or 1 to 4 carbon atoms with ethoxy and particularly methoxy groups being of most interest. Conveniently the total number of carbon atoms in such an alkoxyalkyl group is from 2 to 6, particularly 2 to 4 and especially 2 or 3. Examples of specific substituted groups $R_1$, $R_2$, $R_3$ and $R_4$ are the groups hydroxymethyl, 2-hydroxyethyl and methoxymethyl.

It is generally preferred, however, that where they do not constitute bridged groups $R_1$, $R_2$, $R_3$ and $R_4$ are selected from hydrogen and unsubstituted acyclic aliphatic hydrocarbon groups, especially from the group consisting of hydrogen, methyl, ethyl, n-propyl, isopropyl, allyl and propargyl. Preferably $R_1$ is hydrogen and conveniently either $R_2$ is also hydrogen but $R_3$ and $R_4$ are not or, more usually, $R_3$ is also hydrogen whilst $R_2$ and $R_4$ are either hydrogen or not, for example conveniently being selected from the whole group specified above, but within the proviso given hereinbefore that compounds having n = 0 and $R_1 = R_2 = R_3 = R_4 = H$ or $R_1 = R_2 = R_3 = H$, $R_4 = CH_3$ are excluded. Also of interest, however, are compounds containing a bridging group, particularly a tetramethylene group. Examples of compounds of particular interest are those in which n is 0, $R_1$ and $R_2$ are each hydrogen and $R_3$ and $R_4$ are each methyl, or more particularly n is 0, $R_1$ and $R_3$ are each hydrogen and (a) $R_2$ is hydrogen and $R_4$ is ethyl, n-propyl, isopropyl, allyl or propargyl or (b) $R_2$ is methyl or ethyl and $R_4$ is methyl, ethyl, n-propyl, isopropyl, allyl or propargyl, especially preferred combinations being n = 0, $R_1 = R_2 = H$, $R_3 = R_4 = CH_3$; n = 0, $R_1 = R_2 = R_3 = H$, $R_4 = CH_2OH$ or $CH_2OCH_3$; particularly n = 0, $R_1 = R_3 = H$, $R_2 = R_4 = CH_3$; n = 0, $R_1 = R_3 = H$, $R_2 + R_4 = CH_2CH_2CH_2CH_2$; and especially n = 0, $R_1 = R_2 = R_3 = N$, $R_4 = C_2H_5$.

As indicated previously, the pro-drugs (II) are converted in vivo to the compounds (III). By modification of the nature of the ester groups $CO.OR_5$ it is possible to alter the water solubility, lipophilicity and rate of conversion to the active molecules (III), the methyl, lower hydroxyalkyl and lower alkoxyalkyl esters, for example, having a much greater water solubility than the cyclic diimides which they generate. Thus, the diimide (III) in which n = 0, $R_1 = R_2 = R_3 = H$, and $R_4 = C_2H_5$ has a low water solubility at 20 °C and pH 7.2 (about 0.1% w/v) and is therefore difficult to use parenterally, whereas the corresponding compounds (II) in which $R_5 = CH_3$ or $CH_2CH_2OH$ have a much higher water solubility and can readily be used for parenteral administration. Similarly, increased lipophilicity may be obtained in these pro-drugs by the introduction of, for example, hydrocarbon groupings such as isobutyl or benzyl and this is reflected by relatively high Rf values in a 50% v/v chloroform:ethanol/$SiO_2$ thin layer chromatography system. An increased lipophilicity favours better absorption and changes in tissue distribution. Further, depending upon whether $R_5$ is an electron-withdrawing group, for example benzyl, propargyl or ethoxycarbonylmethyl, or an electron-repelling group, for example isobutyl, hydroxyethyl or methoxyethyl, the rate of conversion of the compound (II) to the corresponding compound (III) can be speeded up or slowed down. Thus, by providing some control over the water solubility, lipophilicity and rate of generation of the active species, the pro-drug compounds of the present invention provide the ability to modify the tissue distribution and pharmacodynamics of the dioxopiperazine protective drugs with consequential therapeutic benefit to the patient.

The group $R_5$ is eliminated under physiological conditions and, although it is possible that enzymic catalysis may be involved in some cases, the elimination will usually occur spontaneously and may be tested for in vitro by incubation of the compound (II) under physiological conditions of pH and temperature (i.e. pH 7.2, 37°C), for example as described in Example 11(B) of UK Patent GB 2173195. Whilst a very wide range of unsubstituted and substituted aliphatic hydrocarbon groups may conveniently be used as the group $R_5$, it has been found that certain groups $R_5$ such as t-butyl groups normally undergo $S_N1$ reactions which do not involve cyclisation so that no significant amount of the desired compound (III) is produced. For this reason, the unsubstituted and substituted aliphatic hydrocarbon groups $R_5$ of use in the present invention preferably contain a bonding carbon atom i.e. that atom linked to the group

$$-O-\overset{\overset{O}{\|}}{C}-,$$

which carries at least one hydrogen atom. Subject to this preference, $R_5$ may conveniently be selected from aliphatic hydrocarbon groups having a maximum of ten carbon atoms which may be either unsubstituted or substituted. Preferences as to the branching, degree of unsaturation and size of these aliphatic hydrocarbon groups are broadly as discussed hereinbefore in relation to $R_1$, $R_2$, $R_3$ and $R_4$. As regards substitution, for example in a substituted alkenyl, alkynyl or particularly alkyl group, this may conveniently involve as substituents one or more groups selected from halogeno (for example fluoro, chloro or bromo), hydroxy, alkoxycarbonyl, benzyloxycarbonyl, cyano, amino (and mono- and di-alkylamino) groups, alkoxy, carboxy and oxo groups. The alkoxy and alkyl groups present as the whole or a part of a substituent in a substituted aliphatic hydrocarbon group are conveniently of one to ten, especially one to four carbon atoms. In general, substituted alkyl groups, especially the alkoxyalkyl groups which may conveniently contain a maximum of ten carbon atoms in total, are of particular interest. Examples of groups containing an oxo substituent are acetonyl and phenacyl, suoh groups providing ketonic esters, particularly $\beta$-keto esters. Also of some particular interest as substituted aliphatic hydrocarbon groups are aralkyl, aralkenyl and aralkynyl groups in which the aromatic part of the group may optionally be substituted by one or more, particularly one or two, substituents selected from halogeno, lower alkyl, lower alkoxy, amino (and mono- and di-alkylamino) and nitro groups or by one methylenedioxy group, and the aliphatic hydrocarbon part of the group (again conveniently having a maximum of ten carbon atoms) may optionally be substituted by one substituent selected from alkoxycarbonyl and cyano. The term 'lower' is used herein to denote a group of 1 to 4 carbon atoms. As regards the aromatic groups, the preferred form of group is an unsubstituted or substituted aromatic hydrocarbon group, particularly a naphthyl or especially a phenyl group, although aromatic heterocyclic groups are also of interest, for example pyridyl groups such as pyrid-2-yl, pyrid-3-yl and pyrid-4-yl.

Among the various aliphatic hydrocarbon groups $R_5$, both unsubstituted and substituted, it is preferred for reasons of ease of synthesis and stability of the compound (II) prior to administration that the bonding carbon atom of the group, as defined hereinbefore, is not linked to any atom which is not hydrogen or carbon and, moreover, conveniently also is not unsaturated, i.e. is not linked to any adjacent atom by a double or particularly a triple bond. Moreover, while the groups, for example alkyl groups, may be substituted by one or more substituents, groups containing one or two substituents are preferred, and conveniently only one substituent in most cases although with some substituent groups, such as alkoxycarbonyl groups, the presence of two substituents may be of value. Those comments also apply to the case of aliphatic hydrocarbon groups substituted by an aryl group which most usually contain two or particularly one aryl group, although groups such as the diphenylmethyl group may be of interest in resisting the esterase activity which occurs in some animal species as discussed hereinafter.

Although substituted aliphatic hydrocarbon groups $R_5$, for example substituted alkyl groups, are of more interest than is the case with $R_1$, $R_2$, $R_3$ and $R_4$, unsubstituted groups, particularly alkyl, alkenyl and alkynyl groups, are also of particular interest.

Specific examples of preferred groups $R_5$ are as follows (the terms ethyl, propyl and butyl used without qualification in the names of the substituted groups as usual indicate a substituted n-alkyl group (and similarly for alkoxy groups present in a substituent) but, except where indicated, without any restriction upon the position of the substituent in the carbon chain of that alkyl group although, as mentioned hereinbefore, substitution upon the bonding carbon atom is generally of lesser interest, substitution upon the terminal carbon of the chain usually being of most interest; in the cases where $R_5$ is a substituted benzyl group, substitution at the $\alpha$-position is specifically indicated and where this is not done the substituent is located on

the ring): methyl, ethyl, n-propyl, n-butyl, isobutyl, allyl, propargyl, benzyl, α-methylbenzyl, α-ethoxycarbonylbenzyl, nitrobenzyl, aminobenzyl, mono- and di-chlorobenzyl, chloro-3,4-methylenedioxybenzyl, mono- and di-methoxybenzyl, mono- and di-methylbenzyl, cinnamyl, methoxyethyl, ethoxyethyl, propoxyethyl, butoxyethyl, hydroxyethyl, hydroxypropyl, hydroxybutyl, ethoxycarbonylmethyl, ethoxycarbonylethyl, ethoxycarbonylpropyl, carboxymethyl, carboxyethyl, carboxypropyl, benzyloxycarbonylmethyl, benzyloxycarbonylethyl, benzyloxycarbonylpropyl, t-butoxycarbonylmethyl, t-butoxycarbonylethyl, t-butoxycarbonylpropyl, di-(ethoxycarbonyl)methyl, cyanomethyl, acetonyl, phenacyl and 3-dimethylaminopropyl.

Particularly preferred compounds according to the present invention are those having the specific combinations of n, $R_1$, $R_2$, $R_3$ and $R_4$ as indicated previously, for example n = 0, $R_1$ = $R_3$ = H, $R_2$ = $R_4$ = $CH_3$; and especially n = 0, $R_1$ = $R_2$ = $R_3$ = H, $R_4$ = $C_2H_5$; whilst $R_5$ is selected from methyl, ethyl, isobutyl, allyl, propargyl, benzyl, α-methylbenzyl, α-ethoxycarbonylbenzyl, o-nitrobenzyl, aminobenzyl, 2,6-dichlorobenzyl, methoxyethyl, ethoxyethyl, propoxyethyl, butoxyethyl, hydroxyethyl, hydroxypropyl, hydroxybutyl, ethoxycarbonylmethyl, di-(ethoxycarbonyl)methyl, benzyloxycarbonylmethyl, ethoxycarbonylpropyl, carboxymethyl and acetonyl (as regards the substituted ethyl and propyl groups particularly those which are terminally substituted). Compounds in which n = 0, $R_1$ = $R_2$ = $R_3$ H, $R_4$ = $CH_2OH$ and $R_5$ is as just defined may have particular value through their enhanced water solubility.

As indicated hereinbefore, when n is 0 and $R_1$ and $R_3$ are each hydrogen but neither $R_2$ nor $R_4$ is hydrogen, the compounds (II) are limited to those in which the groups $R_1$ and $R_4$ are oppositely disposed in the dl or threo configuration (including the enantiomeric forms thereof) rather than adjacently disposed in the meso or erythro configuration, compounds of this latter type being cytotoxic. In other cases where such stereoisomerism can occur the compounds may be in either form. Moreover, compounds in which two of $R_1$ to $R_4$ provide a bridging group may be in the cis or trans form. It will also be appreciated that when the grouping $(CH_2)_n$-$CR_1R_2$-$CR_3R_4$- does not have a centre of symmetry, the compounds can exist in enantiomorphic d and l forms. The invention includes the use of the various different isomers of the compounds except where specifically prohibited. In some cases the optically active d - and l -isomers may have the advantage of significantly higher water solubility than the corresponding racemate and it may also be the case that the biological activity of the compound will differ as between the isomers. The invention does therefore extend to the use of such compounds not only as the dl -racemate but also in a form in which the amount of the compound in either the d or l configuration is greater than that in the l or d configuration, respectively (including amounts in that configuration present in the dl racemate). In particular the compound may be essentially in the form of the d or l isomer, for example being substantially free from (i.e. containing no more than 20% and conveniently no more than 10% of) the d l and l or dl and d isomers. However, where the advantage lies in enhanced solubility of the optically active isomers compared with the racemate, rather than enhanced biological activity for one isomer, any enhancement of the proportion of one isomer should have some effect.

In the literature, Houghton and Williams (Journal of the Chemical Society, Perkin Transactions I, 1982, 2693) describe the preparation of a compound having a structure related to the compound of formula (II) but with one of the specifically excluded combinations, n = 0, $R_1$ = $R_2$ = $R_3$ = $R_4$ = H and $R_5$ = $CH_3$, the copper chelate of the bis-cyclic imide (III, $R_1$ = $R_2$ = $R_3$ = $R_4$ = H) being reacted with an excess of methanol and the copper subsequently removed from the product with hydrogen sulphide. This method works well with methanol but is less successful with ethanol and becomes progressively more difficult with higher alcohols. We have found that it is advantageous to replace the cupric chloride used by Houghton and Williams to make the initial chelate by a cupric salt of a sulphonic acid such as methane sulphonic or isethionic acid. These give more soluble intermediates which lead to more efficient reactions and, after treatment with hydrogen sulphide, to the direct isolation of more water soluble, pharmaceutically acceptable salt forms of the described products. It may sometimes also be advantageous to add about two equivalents of free sulphonic acid, for example methane sulphonic acid, to the initial reaction mixture. Reasonable yields of diesters from higher alcohols such as 2-butoxyethanol may also be obtained by using an ester exchange reaction between the dimethyl ester, activated in the form of the copper chelate and an excess of the appropriate alcohol. Treatment with hydrogen sulphide may again be used to liberate the desired product. The use of other forms of activated ester for this purpose may also be employed.

A more generally useful method, particularly appropriate where the alcohol is not a liquid, or is uneconomic to use in excess or is pH-labile, is neutral esterification using caesium salts and the appropriate halide, which is more reactive than the corresponding alcohol as described for simple N-acyl amino acids by Wang et al , (Journal of Organic Chemistry, 1977, 42 , 1286). In this procedure the appropriate bis-diacid diamide (IV) (prepared as described by Huang et al , Agents and Actions, 1982, 12 , 536) is carefully neutralised with caesium bicarbonate (or caesium carbonate) and a solution, or more usually a suspension, of the dried salt in a neutral aprotic solvent such as dimethylformamide, is treated with a reactive halide

such as benzyl bromide. The reaction is usually complete within a few hours at from 50 to 100°C. Alternative solvents include hexamethylene phosphoramide, dimethylsulphoxide and N-methylpyrrolidone and the caesium salts can generally be replaced by rubidium salts and, in favourable cases where the halide is particularly reactive such as with the benzyl halides, by salts of other metals including sodium or potassium as well as by salts of tertiary amines such as triethyl-amine or 4-dimethylaminopyridine.

Yet another procedure involves the use of an acetal of dimethylformamide of formula $(R_5O)_2CH.N(CH_3)_2$ which is reacted with the appropriate diacid diamide (IV), conveniently by heating the two reactants in a suitable mutual solvent, an excess of the acetal generally being employed. Reaction at 50 to 100°C is usually appropriate, refluxing benzene being suitable as the reaction medium in many cases. This reaction is particularly adapted to the preparation of compounds in which $R_5$ is an unsubstituted aliphatic hydrocarbon group, for example ethyl, methyl, isopropyl, n-propyl, n-butyl etc.

It will be appreciated that the present invention includes a process for the preparation of a compound of formula (II):

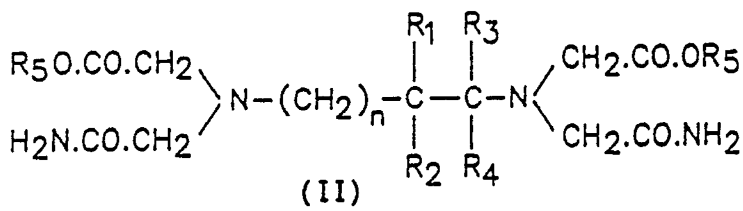

(II)

wherein n is 0, 1 or 2, $R_1$, $R_2$, $R_3$ and $R_4$ are each separately selected from hydrogen and acylic aliphatic hydrocarbon groups having up to a maximum of six carbon atoms and being unsubstituted or substituted or one of $R_1$ and $R_2$ and one of $R_3$ and $R_4$ is hydrogen and the others together are a trimethylene, tetramethylene or pentamethylene bridging group, and $R_5$ is a group such that under physiological conditions $R_5OH$ undergoes elimination with the formation of a 3,5-dioxopiperazinyl ring, but with the provisos that (a) when n = 0 the combinations $R_1 = R_2 = R_3 = R_4 = H$ and $R_1 = R_2 = R_3 = H$, $R_4$ = methyl are excluded and (b) when n is 0 and $R_1$ and $R_3$ are each hydrogen, and each of $R_2$ and $R_4$ is the same or different unsubstituted or substituted aliphatic hydrocarbon group the compound is not in the meso or erythro configuration, and salts thereof formed with a physiologically acceptable inorganic or organic acid, which comprises reacting a compound of formula (IV):

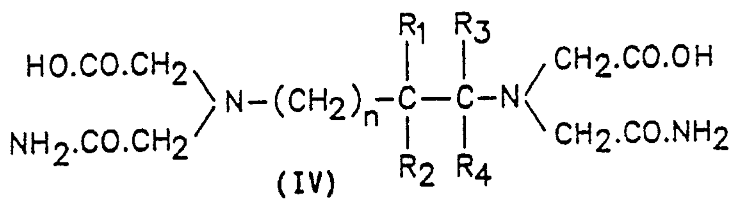

(IV)

or a related compound in which the carboxy groups are in derivative form, including that form in which the carboxy groups are derivatised by the amide groups to form 3,5-dioxopiperazinyl rings, with an alcohol $R_5OH$ or a derivative thereof, where appropriate using a compound of formula (IV) or a related compound as a salt formed with a physiologically acceptable inorganic or organic acid or reacting the compound (II) from the reaction with the alcohol $R_5OH$ or a derivative thereof with such an acid to form a salt.

As discussed hereinbefore, the alcohol $R_5OH$ is preferably a primary or secondary one, i.e. the carbon atom joined to the hydroxy group carries one or two hydrogen atoms, in order to produce a compound (II) in which the bonding carbon atom of the group $R_5$ carries at least one hydrogen atom, which would not be the case with a tertiary alcohol. To obtain compounds (II) of the desired stereochemistry it is most convenient to use a compound (IV) or a related compound having the equivalent stereochemistry. When a d or l isomer is required rather than the dl isomer, however, an alternative to the utilisation of a d or l compound (IV), which is preferred, is to effect a resolution of the compound (II), for example using an appropriate optically active acid to form a mixture of salts of the d and l forms of the compound (II) which are then separated.

The present invention also includes pharmaceutical compositions comprising as an active component a compound of formula (II) as defined hereinbefore, together with a physiologically acceptable diluent or carrier. As indicated, the compounds may be formulated as salts formed with physiologically acceptable

inorganic or organic acids and, when so formulated, it is preferred to use methane sulphonic acid, isethionic acid, tartaric acid or another solubilising acid.

The compounds of formula (II) may be formulated singly, or as a mixture of two or more compounds, for use as pharmaceuticals by a variety of methods. For instance, they may be applied as aqueous, oily (e.g. as a suspension in isopropyl myristate), or in some cases emulsified compositions for parenteral administration and therefore preferably sterile and pyrogen-free. Some of these compounds have rather low solubility in aqueous media and are therefore usually administered in the form of aqueous suspensions containing suitable surface active agents. It will be appreciated that the dosage levels used may vary over quite a wide range especially since certain of the compounds (III) are more active than others and as the rate of formation of these compounds will depend upon the particular nature of the group $R_5$ in the pro-drug (II) which is being used. Moreover, the amount of active compound (III) produced by a given weight of a pro-drug (II) will depend upon the nature of the groups $R_5$ therein and the following discussion is therefore particularly directed to the use of methyl esters. If the group $R_5$ is sufficiently large to increase the molecular weight of the compound (II) significantly beyond that of the corresponding methyl ester then a corresponding increased dosage may well be appropriate. Without commitment to a rigid definition of dosages it may be stated that a daily dosage of active constituent (estimated as the free base), divided if necessary, of from 10 mg to 3 g is proposed for parenteral mammalian use. This dosaage may conveniently be applied as a solution in 500-1000 ml of liquid for intravenous injection by slow infusion, or as a solution or suspension in about 10 ml of liquid by the intramuscular route, or in small volumes subcutaneously. (Parenteral, particularly intravenous, administration is the route preferred for use in conjunction with the anthracycline drugs so that injectable compositions are of especial interest.) More particularly, with many compounds (II) the daily dose for a 70 kg human, administered parenterally, will often be in the range from 100 mg to 500 mg but with the more active compounds it may be less than this (the dose being varied pro rata for humans of a different weight or other mammals). When used in conjunction with an anthracycline drug, where a single administration of the drug and the compound (II) is common, however, higher doses than this may often be emplyed, for example between about 500 mg and about 3 g, with doses of more than this being considered where appropriate in terms of the ratios of compound (II):anthracycline drug as discussed hereinafter.

Where appropriate, the substances may also be compounded for oral administration in dosages which may be similar but may often be somewhat higher, for example in a range from 100 mg to 1 g or even as high as 3 g for the daily dose for a 70 kg human for many compounds (II) but possibly somewhat less than this for the more active compounds. Such oral formulations may particularly take the form of tablets compounded in the presence of conventional solid carrier materials such as starch, lactose, dextrin and magnesium stearate, or of capsules or cachets. Suppositories, pessaries, aerosol and other formulations may also be employed. The compounds may be formulated in unit dosage form, i.e. in discrete portions each containing a unit dose, or a multiple or sub-multiple of a unit dose of the active ingredient.

It will be appreciated that certain formulations of the compounds (II) will tend to cyclise to the compounds (III) on storage if made up in advance. For this reason, although the compounds may conveniently be formulated in advance of their use as a solid composition it will usually be appropriate to prepare certain forms of liquid composition, particularly those containing an aqueous diluent, just prior to their use. Providing such steps are taken to avoid premature cyclisation before administration, however, it will be appreciated from the foregoing discussion that the compounds used may have a very wide range of half lives in vivo .

The pro-drug compounds (II) of the present invention are primarily of value as cardioprotective agents and it should be noted that their potential in such a use extends not only to use in conjunction with drugs having a cardiotoxic side effect, these often being cytotoxic agents such as the anthracycline group of drugs which are of particular value in treating breast cancer, but also extends to pathological conditions where the heart is at risk. The term "anthracycline drug" is used herein to include not only natural and semi-synthetic anthracyclines such as epirubicin, idarubicin, daunorubicin and especially doxorubicin (which names are used herein to include salts of these compounds), but also synthetic anthracyclines such as mitoxantrone. Indeed, the compounds (II) are of value in providing cardioprotection against the cardiotoxic side effect of various compounds containing a moiety

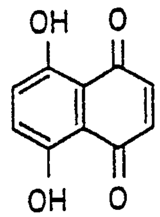

the toxic effect of such compounds being believed to derive from their chelating ability. The compounds (II) also find a secondary use in protection against other toxic effects arising from natural diseases or induction by drugs, for example by various agents which are either toxic as such or when present in the body in excess, such agents including paracetamol (p-hydroxyacetanilide) and various metals such as iron, zinc, cadmium, nickel and lead. In many of these cases, particularly when the toxic agent is a metal, the chelating ability of the compounds (III) produced in vivo by the pro-drugs (II) is often an important factor in achieving the protective effect.

The compounds (II) find most application in the treatment of humans and although they can find veterinary use in certain other mammals such as dogs, rabbits, cattle, and horses, their activity is not expressed in rodents such as rats and mice owing to an esterase activity existing in the plasma thereof which prevents cyclisation of the compounds (II) to the compounds (III).

When used as a cardioprotective agent in the context of a pathological condition where the heart is at risk as a result of that condition the compounds (II) are administered for a period dictated by the existence of this condition. When used in a cardioprotective role in conjunction with a drug having a cardiotoxic side effect, the period of administration will be related to that of the use of the drug which will usually be administered at normal dosage rates and in the usual regimen, of ten parenterally. The compounds (II) may conveniently be administered before, together with or less often after the drug, the choice depending to some extent on the particular drug in question. In the first and third usages both the compound (II) and the drug will each be formulated separately, usually in a conventional manner, for example both being formulated as described above, although the two compositions may be packaged together for ease of sequential administration to the patient. A suitable time lapse between administration of the compound (II) and the drug in either order is quite short, being no more than about 1 to 4 hours, for example 2 hours, and particularly being about 1 hour or somewhat less, depending on the drug in question.

When the compound (II) is administered together with the drug, the two may be formulated separately but it may be preferred to include the compound (II) and the drug in the same composition. Such a pharmaceutical composition may again conveniently take one of the forms described above for compositions containing only the compound (II) and may, if desired, contain more than one compound (II) and/or more than one drug. The present invention thus includes (a) a pharmaceutical composition which comprises a compound of formula (II), as defined hereinbefore, and a drug having a cardiotoxic or other toxic side effect, for example an anthracycline drug, together with a physiologically acceptable diluent or carrier, and also (b) a kit comprising in association a compound of formula (II), as defined hereinbefore, and a drug having cardiotoxic or other toxic side effect.

As indicated, the compounds (II) are of particular interest for use with doxorubicin and the present invention therefore particularly includes a pharmaceutical composition comprising a compound of formula (II) as defined hereinbefore, for example one in which $n = 0$, $R_1 = R_2 = R_3 = H$ and $R_4 = C_2H_5$, and doxorubicin, together with a physiologically acceptable diluent or carrfier.

In instances where a series of doses of the drug is administered it may not be necessary for each administration of the drug to be made concomitantly with, or at the interval given above after or before the administration of the compound (II). It may be possible to administer the compound (II) alone or together with the drug, followed by one or more repeated spaced doses of the drug alone or, more often, in view of the more rapid metabolisation of the compound (II), to administer the drug alone or together with the compound (II), followed by one or more repeated spaced doses of the compound (II) alone. If the treatment with the drug is continued over an extended period repeat doses of the compound (II) are also likely to be required and one possible regimen would involve the administration of the drug and compound (II) together on certain occasions followed by the compound (II) alone on others.

As regards the relative amounts of the compound (II) and a drug to be used, this will depend on both the particular compound (II) and the drug used and the regimen of use, a good indication being provided, however, by the dosages indicated hereinbefore for the compounds (II) and the conventional doses used for the drug. However, some additional comments may be made concerning the proportions of compound or compounds (II) to anthracycline which are used either singly or together in a pharmaceutical composition

containing both a compound or compounds (II) and an anthracycline drug. Thus, by way of guidance it may be stated that a dose ratio of between 5:1 to 20:1 or even 25:1 w/w of compound or compounds (II) to drug, especially about 10:1 w/w, is often suitable. By way of further guidance, it may be mentioned that a normal single dosage of doxorubicin is in the range of about 0.75 to 2 mg/kg, i.e. about 50 to 150 mg, for a 70 kg human being, but that the use of the compounds (II) is intended to enable some increase in the dosage, for example to 4 or 5 mg/kg, if desired, in order to enhance the anti-cancer effect of the doxorubicin whilst its cardiotoxic side effects are controlled by the presence of the compound (II).

The exact dosage of an anthracycline drug such as doxorubicin which is used will depend on whether it is given with other anti-tumour agents. Thus anthracycline drugs are often given together with one or more of other such agents, for example fluorouracil and cyclophosphamide and, where desired, a pharmaceutical composition containing a compound or compounds (II) and an anthracycline drug can contain other such anti-tumour agents. Moreover, it may be advantageous to administer a calcium supplement together wih the compounds (II), this usually being administered separately.

When used as a protective agent against the toxic effect of a metal, or an excess thereof, or against the toxic effect of paracetamol, the compounds (II) may be used protectively before occurrence of the toxicity or following occurrence of the toxicity. It may even be possible to formulate the compound (II) with paracetamol in order automatically to counter the effect of an overdose thereof. Broadly similar dosage levels may be used to those described hereinbefore although differences may arise as to whether the toxic effect is acute, as for example is usually the case following an overdose of paracetamol, or chronic, as will often be the case with conditions such as iron overload: higher dosages over a shorter period being indicated in the former type of case as compared with the latter.

Other forms of protection include the use of the compounds (II) in conjunction with any condition which is either "naturally occurring" or drug induced where free radical damage occurs (this may also be involved in some of the conditions described hereinbefore such as an anthracycline drug-induced damage), for example in reducing the diabetogenic effect of drugs such as alloxan which generate free hydroxyl radicals. The compounds (II) may once again be used in a broadly similar manner as when employed in cardioprotection, including formulation together with the drug, and the dosage levels used.

The present invention thus includes a method of providing protection against a toxic effect on the body, particularly a cardiotoxic effect, which comprises administering to a patient in need thereof a therapeutically effective amount of a compound (II) as defined hereinbefore. Furthermore the invention includes the use of a compound (II) in the manufacture of a medicament for use in providing protection against a toxic effect on the body.

The present invention is illustrated by the following Examples. Further exemplification of preparative procedures is provided by the Examples of UK Patent No. GB 2173195 which, although directed to the preparation of cytotoxic compounds, do involve reactions of a similar chemical nature.

## EXAMPLES

### Example 1 : Preparation of NN′-Dimethoxycarbonylmethyl-NN′-diaminocarbonylmethyl-1,3-diaminopropane dimethanesulphonate

A mixture of 1,3-bis-(3,5-dioxopiperazin-1-yl)propane (0.1 moles), cupric methanesulphonate (0.1 moles) (prepared from equivalent amounts of cupric acetate and methanesulphonic acid) and dry methanol (500 ml) is stirred and heated under reflux for 48 hours. The reaction mixture is then evaporated to dryness, the residue taken up in water (200 ml), and the solution satud with hydrogen sulphide and then filtered. The resultant colourless solution is evaporated to dryness to yield a white solid which is recrystallised from methanol (100 ml) containing a little water. Concentration of the mother liquors to 50 ml followed by dilution with acetone (500 ml) gives on cooling a further crop of solid providing a total yield of 43% of NN′-Dimethoxycarbonylmethyl-NN′-diaminocarbonylmethyl-1,3-diaminopropane dimethanesulphonate having m.p. 157-159°C.

### Example 2 : Formulation of compounds

(A) Tablets of the following composition are prepared:

| | mg/tablet |
|---|---|
| Compound of Example 1 (micronised) | 250 |
| 'Avicel' (microcrystalline cellulose)* | 38 |
| polyvinylpyrrolidone | 3 |
| alginic acid | 6 |
| magnesium stearate | 3 |

* 'Avicel' is a Registered Trade Mark or Service Mark.

The compound of Example 1 is mixed with 'Avicel' and polyvinylpyrrolidone is added, dissolved in sufficient industrial methylated spirits (74° OP) to produce a mass suitable for granulating. The mass is granulated through a 20 mesh sieve and the resultant granules are dried at a temperature not exceeding 50°C. The dried granules are passed through a 20 mesh sieve and the alginic acid and magnesium stearate are then added and mixed with the granules. The product is compressed into tablets each weighing 300 mg on 3/8 inch flat bevelled edge divided punches.

(B)Tablets of the following composition are prepared:

| | mg/tablet |
|---|---|
| Compound of Example 1 (micronised) | 250 |
| 'Avicel' (microcrystalline cellulose) | 134 |
| polyvinylpyrrolidone | 4 |
| alginic acid | 8 |
| magnesium stearate | 4 |

The tablets are prepared by essentially the same procedure as described in (A) and are compressed at a tablet weight of 400 mg on 7/16 inch flat bevelled edge punches.

(C) Tablets of the following composition are prepared:

| | mg/tablet |
|---|---|
| Compound of Example 1 (micronised) | 250 |
| lactose (300 mesh) | 19 |
| maize starch | 15 |
| gelatine | 10 |
| magnesium stearate | 6 |

The tablets are prepared by mixing the compound of Example 1 with lactose and half the total quantity of maize starch required, and adding to the mass a 5% solution of gelatine in water. The product is granulated through a 16 mesh sieve, and the resultant granules are dried to constant weight at a temperature not exceeding 50°C. The dried granules are passed through a 20 mesh sieve and mixed with magnesium stearate and the remainder of the maize starch. The product is compressed at a 300 mg tablet weight on 3/8 inch flat bevelled edge divided punches.

## Claims

1. A compound of formula (II):

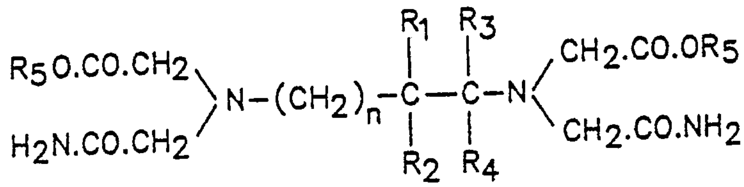

(II)

wherein n is 0, 1 or 2, $R_1$, $R_2$, $R_3$ and $R_4$ are each separately selected from hydrogen, unsubstituted acyclic aliphatic hydrocarbon groups having a maximum of six carbon atoms and $C_{1-6}$ alkyl groups substituted by a hydroxy group or by a $C_{1-6}$ alkoxy group, or one of $R_1$ and $R_2$ and one of $R_3$ and $R_4$ is hydrogen and the others together are a trimethylene, tetramethylene or pentamethylene bridging group, and $R_5$ is a group such that under physiological conditions $R_5OH$ undergoes elimination with the formation of a 3,5-dioxopiperazinyl ring, but with the provisos that (a) when n = 0 the combinations $R_1 = R_2 = R_3 = R_4 = H$ and $R_1 = R_2 = R_3 = H$, $R_4$ = methyl are excluded and (b) when n is 0, $R_1$ and $R_3$ are each hydrogen, and each of $R_2$ and $R_4$ is other than hydrogen the compound is in other than the meso or erythro configuration, and salts thereof formed with a physiologically acceptable inorganic or organic acid.

2. A compound according to Claim 1, in which $R_1$, $R_2$, $R_3$ and $R_4$ are each separately selected from hydrogen and $C_{1-4}$ alkyl, $C_{2-4}$ alkenyl and $C_{2-4}$ alkynyl groups or one of $R_1$ and $R_2$ and one of $R_3$ and $R_4$ is hydrogen and the others are a trimethylene, tetramethylene or pentamethylene bridging group.

3. A compound according to Claim 2, in which $R_1$, $R_2$, $R_3$ and $R_4$ are each separately selected from hydrogen, methyl, ethyl, n-propyl, isopropyl, allyl and propargyl or one of $R_1$ and $R_2$ and one of $R_3$ and $R_4$ is hydrogen and the others are tetramethylene.

4. A compound according to any of Claims 1 to 3, in which n is 0.

5. A compound according to Claim 1, in which n is 0, $R_1$ and $R_2$ are each hydrogen and $R_3$ and $R_4$ are each methyl, or n is 0, $R_1$ and $R_3$ are each hydrogen and (a) $R_2$ is hydrogen and $R_4$ is ethyl, n-propyl, isopropyl, allyl or propargyl or (b) $R_2$ is methyl or ethyl and $R_4$ is methyl, ethyl, n-propyl, isopropyl, allyl or propargyl.

6. A compound according to Claim 1, in which $n = 0$, $R_1 = R_2 = H$, $R_3 = R_4 = CH_3$; $n = 0$, $R_1 = R_2 = R_3 = H$, $R_4 = CH_2OH$ or $CH_2OCH_3$; $n = 0$, $R_1 = R_3 = H$, $R_2 + R_4 = CH_2CH_2CH_2CH_2$; or $n = 0$, $R_1 = R_2 = R_3 = H$, $R_4 = C_2H_5$.

7. A compound according to Claim 1, in which $n = 0$, $R_1 = R_2 = R_3 = H$, $R_4 = C_2H_5$.

8. A compound according to any of Claims 1 to 7, in which $R_5$ is an unsubstituted aliphatic hydrocarbon group having a maximum of ten carbon atoms, a substituted aliphatic hydrocarbon group which is (a) an aliphatic hydrocarbon group having a maximum of ten carbon atoms which is substituted by one or more groups selected from halogeno, hydroxy, alkoxycarbonyl, benzyloxycarbonyl, cyano, amino, mono- and di-alkylamino, alkoxy, carboxy and oxo groups, or (b) an aralkyl, aralkenyl or aralkynyl group in which the aromatic part of the group may optionally be substituted by one or more substituents selected from halogeno, lower alkyl, lower alkoxy, amino, mono- and di-alkylamino, and nitro groups or by one methylenedioxy group, and the aliphatic hydrocarbon part of the group, which has a maximum of ten carbon atoms, may optionally be substituted by one substituent selected from alkoxycarbonyl and cyano.

9. A compound according to Claim 8, in which $R_5$ is a $C_{1-4}$ alkyl or $C_{2-4}$ alkenyl or alkynyl group, or a $C_{1-4}$ alkyl group substituted either as described under (a) or as described under (b) with the aromatic part of the group being a phenyl group or a substituted phenyl group.

10. A compound according to Claim 9, in which $R_5$ is methyl, ethyl, n-propyl, n-butyl, isobutyl, allyl, propargyl, benzyl, α-methylbenzyl, α-ethoxycarbonylbenzyl, nitrobenzyl, aminobenzyl, mono- and dichlorobenzyl, chloro-3,4-methylenedioxybenzyl, mono-and di-methoxybenzyl, mono- and di-methylbenzyl, cinnamyl, methoxyethyl, ethoxyethyl, propoxyethyl, butoxyethyl, hydroxyethyl, hydroxypropyl, hydroxybutyl, ethoxycarbonylmethyl, ethoxycarbonylethyl, ethoxycarbonylpropyl, carboxymethyl, carboxyethyl, carboxypropyl, benzyloxycarbonylmethyl, benzyloxycarbonylethyl, benzyloxycarbonylpropyl, t-butoxycarbonylmethyl, t-butoxycarbonylethyl, t-butoxycarbonylpropyl, di-(ethoxycarbonyl)methyl, cyanomethyl, acetonyl, phenacyl or 3-dimethylaminopropyl.

11. A compound according to Claim 1, in which $n = 0$, $R_1 = R_3 = H$, $R_2 = R_4 = CH_3$; or $n = 0$, $R_1 = R_2 = R_3 = H$, $R_4 = C_2H_5$; and $R_5$ is selected from methyl, ethyl, isobutyl, allyl, propargyl, benzyl, α-methylbenzyl, ethoxycarbonylbenzyl, o-nitrobenzyl, aminobenzyl, 2,6-dichlorobenzyl, methoxyethyl, ethoxyethyl, propoxyethyl, butoxyethyl, hydroxyethyl, hydroxypropyl, hydroxybutyl, ethoxycarbonylmethyl, di-(ethoxycarbonyl)methyl, benzyloxycarbonylmethyl, ethoxycarbonylpropyl, carboxymethyl and acetonyl.

12. A process for the preparation of a compound of formula (II) as defined in Claim 1 and salts thereof formed with a physiologically acceptable inorganic or organic acid which comprises reacting a compound of formula (IV)

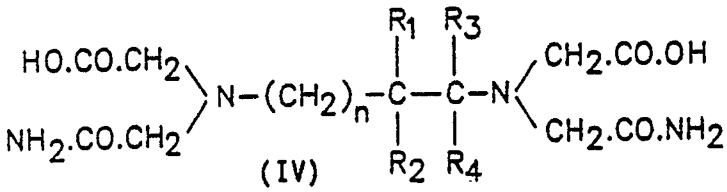

(IV)

or a related compound in which the carboxy groups are in derivative form, including that form in which the carboxy groups are derivatised by the amide groups to form 3,5-dioxopiperazinyl rings, with an alcohol $R_5OH$ or a derivative thereof, where appropriate using a compound of formula (IV) or a related compound as a salt formed with a physiologically acceptable inorganic or organic acid or reacting the compound (II) from the reaction with the alcohol $R_5OH$ or a derivative thereof with such an acid to form a salt.

13. A process according to Claim 12, in which the compound of formula (IV) is reacted with a dimethylformamide acetal of formula $(R_5O)_2CH.N(CH_3)_2$.

14. A process according to CLaim 12, in which the compound of formula (IV) or related compound is reacted in the form of a metal complex or a metal salt of the compound, the metal subsequently being removed.

15. A process according to Claim 12, in which the caesium or rubidium salt of the compound of formula (IV) is reacted with a halide $R_5X$, X being a halogeno group.

16. A process according to Claim 12, in which the copper complex of a related compound wherein the carboxy groups are derivatised by the amide groups to form 3,5-dioxopiperazinyl rings is reacted with an alcohol $R_5OH$.

17. A process according to Claim 12, in which the copper complex of a related compound wherein the carboxy groups are in the form of a methyl ester giving a compound of formula (II) in which $R_5$ is methyl is reacted with an alcohol $R_5OH$ wherein $R_5$ is other than methyl.

18. A pharmaceutical composition comprising a compound of formula (II) as defined in any of Claims 1 to 11 together with a physiologically acceptable diluent or carrier.

19. A pharmaceutical composition according to Claim 18 which further comprises an anthracycline drug.

20. A pharmaceutical composition according to Claim 19, in which the anthracycline drug is doxorubicin.

21. A compound of formula (II) as defined in any of Claims 1 to 11 for use in therapy.

22. The use of a compound of formula (II) as defined in any of Claims 1 to 11 for use in the manufacture of a medicament for use as a cardioprotective agent.

23. A method for the treatment of a patient in need of cardioprotection or in need of protection against the toxic effects of paracetamol or of free radicals which comprises administering to said patient a therapeutically effective amount of a compound of formula (II) as defined in any of Claims 1 to 11.

European Patent Office

# PARTIAL EUROPEAN SEARCH REPORT

which under Rule 45 of the European Patent Convention shall be considered, for the purposes of subsequent proceedings, as the European search report

Application number: EP 90 30 7686

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | GB-A-2 173 195 (NATIONAL RESEARCH DEVELOPMENT CORP.) * Examples; claims * | 1-22 | C 07 C 237/06<br>A 61 K 31/22 |
| A | JOURNAL OF THE CHEMICAL SOCIETY, PERKIN TRANSACTIONS I, no. 11, 1982, pages 2693-2696; R.P. HOUGHTON et al.: "Synthesis of bis(imides) and bis(half amides) of NN'-ethylenebis(iminodiacetic acid)" | | |

TECHNICAL FIELDS SEARCHED (Int. Cl.4): C 07 C 237/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 8-11

Claims searched incompletely: 1-7,12-22

Claims not searched: 23

Reason for the limitation of the search:

Claims 1-7,12-22:
The definition for the $R_5$ moiety in claim 1 is unclear. The search was thus performed on examples and on claims where a clear definition of $R_5$ is given (Art. 84 EPC).

Method for treatment of the human or animal body by surgery or therapy (See art. 52(4) of the European Patent Convention) (Claim 23)

| Place of search | Date of completion of the search | Examiner |
|---|---|---|
| THE HAGUE | 10-10-1990 | ZAROKOSTAS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document